(19) Europäisches Patentamt
European Patent Office
Office européen des brevets

(11) Publication number: **0 318 056 A2**

(12) # EUROPEAN PATENT APPLICATION

(21) Application number: 88119835.2

(22) Date of filing: 28.11.88

(51) Int. Cl.4: C12P 17/18 , C07D 487/04 , C07D 471/04 , //(C07D487/04, 209:00,209:00),(C07D471/04, 221:00,209:00)

(30) Priority: 27.11.87 JP 297476/87
19.04.88 JP 94544/88

(43) Date of publication of application:
31.05.89 Bulletin 89/22

(84) Designated Contracting States:
DE FR GB

(71) Applicant: MEIJI SEIKA KAISHA LTD.
4-16 Kyobashi 2-chome
Chuo-ku Tokyo(JP)

(72) Inventor: Ohba, Kazunori c/o MEIJI SEIKA KAISHA, LTD.
Pharmaceutical Research Lab. 760, Morooka-cho
Kohoku-ku Yokohama-shi Kanagawa(JP)
Inventor: Watabe, Hiroomi c/o MEIJI SEIKA KAISHA, LTD.
Pharmaceutical Research Lab. 760, Morooka-cho
Kohoku-ku Yokohama-shi Kanagawa(JP)
Inventor: Nagasawa, Mieko c/o MEIJI SEIKA KAISHA, LTD.
Pharmaceutical Research Lab. 760, Morooka-cho
Kohoku-ku Yokohama-shi Kanagawa(JP)
Inventor: Sakakibara, Shiro c/o MEIJI SEIKA KAISHA, LTD.
Pharmaceutical Research Lab. 760, Morooka-cho
Kohoku-ku Yokohama-shi Kanagawa(JP)
Inventor: Shomura, Takashi c/o MEIJI SEIKA KAISHA, LTD.
Pharmaceutical Research Lab. 760, Morooka-cho
Kohoku-ku Yokohama-shi Kanagawa(JP)
Inventor: Sezaki, Masaji c/o MEIJI SEIKA KAISHA, LTD.
Pharmaceutical Research Lab. 760, Morooka-cho
Kohoku-ku Yokohama-shi Kanagawa(JP)
Inventor: Kondo, Shinishi c/o MEIJI SEIKA KAISHA, LTD.
Pharmaceutical Research Lab. 760, Morooka-cho
Kohoku-ku Yokohama-shi Kanagawa(JP)

(74) Representative: Wilhelms, Rolf E., Dr. et al
WILHELMS, KILIAN & PARTNER

EP 0 318 056 A2

Patentanwälte Eduard-Schmid-Strasse 2
D-8000 München 90(DE)

(54) **Novel antibiotics and process for preparing the same.**

(57) Novel substances exhibiting antimicrobial activity and antitumor activity, and a process for producing them by cultivating a microorganism belonging to the genus Streptomyces and capable of producing them are disclosed.

# NOVEL ANTIBIOTICS AND PROCESS FOR PREPARING THE SAME

## FIELD OF THE INVENTION

This invention relates to novel antibiotics and to a process for preparing the same. More particularly, it relates to novel antibiotics (hereinafter designated as SF2582 A, B, and C) which exhibit antitumor activity and to a process for preparing them by cultivation of a microorganism belonging to the genus Streptomyces.

## BACKGROUND OF THE INVENTION

A known compound which is similar to the antibiotic substances according to the present invention in physiochemical properties is CC-1065 as reported in D.G. Martin et al., J. Antibiotics, Vol. 34, 1119-1125 (1981). The antibiotic substances according to the present invention have been confirmed to clearly differ from CC-1065 in physiochemical properties and thereby to be novel antimicrobial substances.

A number of antimicrobial and antitumor substances have hitherto been reported, and some of them have been put into practical use as antimicrobials or anticancer agents. However, there are many unsolved problems in their application to the field of chemotherapy.

## SUMMARY OF THE INVENTION

One object of this invention is to provide novel and useful antimicrobial and anticancer substances.

Another object of this invention is to provide a process for preparing such substances from microorganisms.

The inventors have isolated a number of microorganisms from the soil and searched substances produced therefrom. As a result, they have found that a certain kind of microorganism is capable of producing antibiotics exhibiting strong antimicrobial and antitumor activities. Further investigations have proved that these antibiotics are novel substances and produce excellent treating effects on experimentally induced tumors in animals. The present invention has been completed based on these findings.

## BRIEF DESCRIPTION OF THE ACCOMPANYING DRAWINGS

Figure 1 shows a UV Spectrum of SF2582 A in a methanol solution.
Figure 2 shows an IR Spectrum of SF2582 A in a KBr tablet.
Figure 3 shows a $^1H$ NMR Spectrum (400 MHz) of SF2582 A in $CDC\ell_3$.
Figure 4 shows a $^{13}C$ NMR Spectrum (100 MHz) of SF2582 A in $CDC\ell_3$.
Figure 5 shows a UV Spectrum of SF2582 B in a methanol solution.
Figure 6 shows an IR Spectrum of SF2582 B in a KBr tablet.
Figure 7 shows a $^1H$ NMR Spectrum (400 MHz) of SF2582 B in $CDC\ell_3$.
Figure 8 shows a $^{13}C$ NMR Spectrum (100 MHz) of SF2582 B in $CDC\ell_3$.
Figure 9 shows a UV Spectrum of SF2582 C in a methanol solution.
Figure 10 shows an IR Spectrum of SF2582 C in a KBr tablet.
Figure 11 shows a $^1H$ NMR Spectrum (400 MHz) of SF2582 C in DMSO-$d_6$.
Figure 12 shows a $^{13}C$ NMR Spectrum (400 MHz) of SF2582 C in DMSO-$d_6$.

## DETAILED DESCRIPTION OF THE INVENTION

SF2582 A, B, and C according to the present invention have the following physiochemical properties.

[I] SF2582 A

represented by the formula (A):

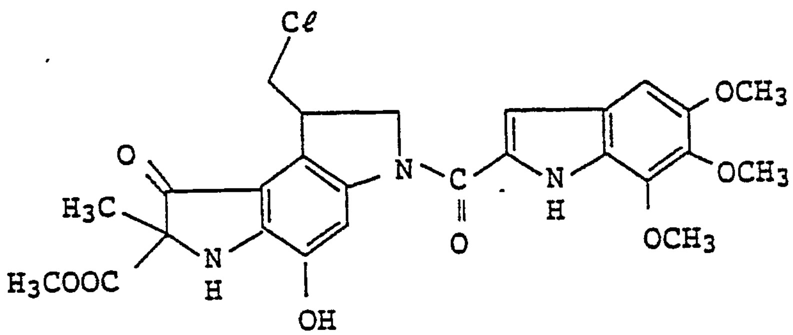

(A)

1) Appearance: yellowish orange amorphous powder

2) Melting Point: 235 - 237° C (decomp.)

3) Molecular Formula: $C_{26}H_{26}N_3O_8Cl$ [determined by high resolution mass spectrometry of a monoacetyl derivative; found: 585.1492; calcd. for $C_{28}H_{28}N_3O_9Cl$: 585.1512]

4) Mass Spectrum: SI-MS m/z 544 $(M+1)^+$
FD-MS m/s 543 $(M)^+$

5) Specific Rotation: $[\alpha]_D^{22}$ -51° (c = 0.2, methanol)

6) UV Spectrum: as shown in Fig. 1
$\lambda_{max}$ (nm) ($E^{1\%}_{1cm}$) (in methanol solution): 208 (729), 245 shoulder (360), 298 (391), 337 (580), 432 (77)

7) IR Spectrum: as shown in Fig. 2

8) $^1$H NMR Spectrum (400 MHz, $CDCl_3$): as shown in Fig. 3

9) $^{13}$C NMR Spectrum (100 MHz, $CDCl_3$): as shown in Fig. 4 $\delta$ (ppm): 196.6 (s), 169.5 (s), 160.5 (s), 150.2 (s), 150.1 (s), 144.1 (s), 140.7 (s), 138.6 (s), 137.6 (s), 129.0 (s), 125.9 (s), 123.4 (s), 119.5 (s), 115.5 (d), 112.4 (s), 107.8 (d), 97.7 (d), 71.1 (s), 61.5 (q), 61.2 (q), 56.3 (q), 54.9 (t), 53.3 (q), 46.3 (t), 42.2 (d), 21.9 (q)

10) Solubility: soluble in methanol, ethyl acetate, acetone, chloroform, or dimethyl sufoxide; insoluble in water or haxane

11) Thin Layer Chromatography (on silica gel plates produced by E. Merck, Darmstadt):

| Developing Solvent | Rf |
|---|---|
| chloroform-methanol (20:1) | 0.52 |
| benzene-acetone (2:1) | 0.48 |
| toluene-acetone (2:1) | 0.45 |

12) Color Reaction: positive with 10% solfuric acid reagent, Lemieux reagent, Greig-Leaback reagent, and molybdate-sulfuric acid reagent; negative with ninhydrin reagent

[II] SF2582 B:

represented by the formula (B):

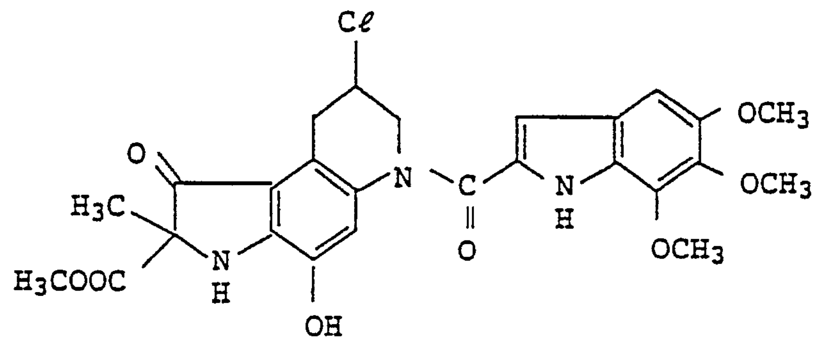

(B)

1) Appearance: yellowish orange amorphous powder

2) Melting Point: 173 - 175 °C (decomp.)

3) Molecular Formula: $C_{26}H_{26}N_3O_8Cl$ [determined by high resolution mass spectrometry of a monoacetyl derivative; found: 585.1519; calcd. for $C_{28}H_{28}N_3O_9Cl$: 585.1512]

4) Mass Spectrum: SI-MS 544 $(M+1)^+$
EI-MS 543 $(M)^+$

5) Specific Rotation: $[\alpha]_D^{22}$ -128° (c=0.2, methanol)

6) UV Spectrum: as shown in Fig. 5
$\lambda_{max}$ (nm) ($E^{1\%}_{1cm}$) (in methanol solution): 210 (991), 320 (563), 400-410 (99)

7) IR Spectrum: as shown in Fig. 6

8) $^1$H NMR Spectrum (400 MHz, $CDCl_3$): as shown in Fig. 7

9) $^{13}$C NMR Spectrum (100 MHz, $CDCl_3$): as shown in Fig. 8 $\delta$ (ppm): 196.8 (s), 169.6 (s), 164.7 (s), 151.6 (s), 150.0 (s), 141.6 (s), 140.2 (s), 138.8 (s), 129.1 (s), 128.8 (s), 125.9 (s), 123.0 (s), 118.0 (d), 116.7 (s), 116.6 (s), 108.1 (d), 97.6 (d), 71.0 (s), 61.4 (q), 61.1 (q), 56.2 (q), 53.6 (d), 53.4 (q), 52.5 (t), 33.1 (t), 21.8 (q)

10) Solubility: soluble in methanol, ethyl acetate, acetone, chloroform, or dimethyl sufoxide; insoluble in water or haxane

11) Thin Layer Chromatography (on silica gel plates produced by E. Merck, Darmstadt):

| Developing Solvent | Rf |
|---|---|
| chloroform-methanol (20:1) | 0.50 |
| benzene-acetone (2:1) | 0.45 |
| toluene-acetone (2:1) | 0.44 |

13) Color Reaction: positive with 10% solfuric acid reagent, Lemieux reagent, Greig-Leaback reagent, and molybdate-sulfuric acid reagent; negative with ninhydrin reagent

[III] SF2582 C:

represented by the formula (C)

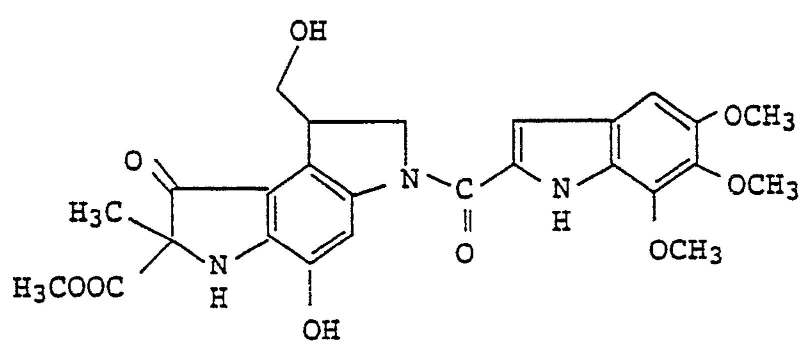

(C)

1) Appearance: orange crystalline powder

2) Melting Point: > 240° C

3) Elementary Analysis: C 59.24%, H 5.12%, N 7.98%

4) Molecular Formula: $C_{26}H_{27}N_3O_9$

5) Mass Spectrum: EI-MS m/z 525 ($M^+$)

6) Specific Rotation: $[\alpha]_D^{22}$ -54° (c = 0.1, methanol)

7) UV Spectrum: as shown in Fig. 9

$\lambda_{max}$ (nm) ($E_{1\,cm}^{1\,\%}$) (in methanol solution): 208 (845), 245 shoulder (450), 297 (473), 336 (690), 432 (93)

8) IR Spectrum: as shown in Fig. 10

9) $^1H$ NMR Spectrum (400 MHz, DMSO-$d_6$): as shown in Fig. 11

10) $^{13}C$ NMR Spectrum (100 MHz, DMSO $d_6$): as shown in Fig. 12

δ (ppm): 198.1 (s), 169.5 (s), 159.6 (s), 150.5 (s), 149.3 (s), 143.1 (s), 139.9 (s), 139.2 (s), 136.5 (s), 131.4 (s), 125.3 (s), 123.4 (s), 120.0 (s), 114.5 (s), 111.4 (d), 105.9 (d), 98.2 (d), 70.1 (s), 62.6 (t), 61.3 (q), 61.1 (q), 56.1 (q), 54.1 (t), 52.8 (q), 42.5 (d), 20.3 (q)

11) Solubility: easily soluble in pyridine or dimethyl sulfoxide; slightly soluble in methanol; sparingly soluble in chloroform, hexane, ethyl acetate, or water

12) Thin Layer Chromatography (on silica gel plates produced by E. Merck, Darmstadt):

| Developing Solvent | Rf |
|---|---|
| chloroform-methanol (10:1) | 0.38 |
| toluene-acetone (1:1) | 0.62 |

13) Color Reaction: positive with 10% sulfuric acid reagent, Lemieux reagent, Greig-Leaback reagent, or molybdate-sulfuric acid reagent; negative with ninhydrin reagent

The microorganisms capable of producing SF2582 A, B, and C include strain SF2582 isolated from the soil of Sagamihara City, Kanagawa, Japan.

Strain SF2582 has the following microbiological properties:

I. Morphology:

Vegetative mycelia extend long and branch. Aerial mycelia grow on starch agar, oatmeal agar, sucrose nitrate agar, etc. with abundant spore formation. Branching of the aerial hyphae is simple and no whirl is found. Spore chain at the top of the aerial hyphae mostly have a closed spiral form. According to electron microscopical observation, spores have a cyclindrical form, a size of 0.7 to 0.9 x 1.0 to 1 4 µm, and a smooth surface, and usually about 20 spores are linked. Sporangia, motile spores or sclerotia are not observed.

II. Growth on Various Media:

Growth of strain SF2582 on various media is shown in Table 1 below. The observations were made after culturing at 28° C for 2 to 3 weeks. The color standards in the parentheses are in accordance with Color Harmony Manual of Container Corporation of America.

TABLE 1

| Medium | Growth (Color of Reverse Side) | Aerial Hyphae | Soluble Pigment |
|---|---|---|---|
| sucrose-nitrate agar | fair (colorless) | abundant, beige grown (3ig) | nil |
| glucose-asparagine agar | fair to good (grayish yellow) | fair, beige (3ge) | nil |
| glycerol-asparagine agar | faint (grayish yellow) | fair, grayish yellow (2gc) | nil |
| malic acid-calcium agar | fair (ivory) | poor, white to grayish white (2dc) | nil |
| starch agar | fair to good (grayish yellow) | fair to abundant, gray (2ih) | nil |
| oatmeal agar | fair to good (grayish grown yellow) | abundant, beige grown (3ig) | nil |
| yeast-malt agar | fair to good (pale grown) | poor to fair, grayish white (2dc) to gray (2ih) | nil |
| tyrosine agar | faint to fair (yellowish brown) | fair, beige (3ge) | pale purple |
| nutrient agar | faint (yellowish brown) | nil | nil |
| Bennett agar | fair to good (pale yellow) | poor, grayish white (2dc) | nil |

III. Physiological Properties:

1) Growth Temperature: grown at 15 to 38° C, optimumly 26 to 30° C, on a yeast-malt agar medium
2) Liquefaction of Gelatin: positive
3) Hydrolysis of Starch: positive
4) Reduction of Nitrate: negative
5) Peptonization of Skimmed Milk: positive Coagulation of Skimmed Milk: negative
6) NaCℓ tolerance: grown in 7% NaCℓ-containing media but not grown in 10% NaCℓ-containing media.
7) Formation of Melanin-like Pigment: negative

IV. Utilization of Carbon Source (ISP No. 9 medium):

1) D-Glucose, glycerol, D-xylose, L-arabinose, D-mannitol, D-fructose, myo-innositol, and L-rhamnose are utilized.
2) Sucrose is not utilized.
3) Utilization of raffinose is doubtful.

V. Cell Wall Composition:

Analysis by the method of Backer et al., Appl. Microbial., Vol. 13, 236 (1965) revealed that diaminopimelic acid in the cell wall composition is of LL form.

From the above properties, it is reasonable to consider that strain SF2582 belongs to the genus Streptomyces of actinomycetes. The inventors designated strain SF2582 as Streptomyces sp. SF2582. Strain SF2582 is deposited in Agency of Fermentation Research Institute, Japan under the deposit number FERM BP-2087 in accordance with Budapest treaty.

Strain SF2582 is liable to variation in properties as is the case of other actinomycetes. Therefore,

microorganisms which can be used in the present invention include not only strain SF2582 per se but all other strains derived therefrom and capable of producing SF2582 A, B, or C, such as spontaneous or induced mutants, zygotes, and genetic recombinants originating from strain SF2582.

The strain is cultured in a medium containing nutrients utilizable by general microorganisms. Known nutrient sources conventionally employed for cultivation of actinomycetes can be used. Specific examples of the nutrient sources are carbon sources, e.g., glucose, maltose syrup, dextrin, starch, molasses, and animal and vegetable oils; and nitrogen sources, e.g., soybean flour, wheat germ, corn steep liquor, cotton seedmeal, meat extract, peptone, yeast extract, ammonium sulfate, sodium nitrate, and urea. If desired, the medium can contain inorganic salts capable of providing a sodium ion, a potassium ion, a calcium ion, a magnesium ion, a cobalt ion, a chlorine ion, a phosphate ion, a sulfate ion or other ions. Further, organic or inorganic substances effective to assist cell growth and to accelerate production of SF2582 A, B or C can be added to the medium appropriately.

The cultivation can be suitably effected under aerobic conditions, and most suitably effected under aerobic conditions, and most suitably by submerged aeration culture. A suitable cultivation temperature is from 26 to 30 °C and, in most cases, around 28 °C. The production of SF2582 A, B, or C varies depending on the medium or cultivation conditions. Usually, the accumulation reaches the maximum in 2 to 7 days by shake culture or tank culture when the accumulated amount in the culture reaches the maximum, cultivation is ceased, and the desired substance is isolated from the culture and purified.

Isolation of SF2582 A, B or C from the culture can be carried out by usual means taking advantage of the properties of the desired substance, such as filtration, solvent extraction, adsorption or partition column chromatography, gel filtration, dialysis, precipitation, or the like technique, either alone or in combination thereof. For example, SF2582 A, B, and C can be extracted with acetone-water or methanol-water from the fungus body. The aqueous extract is then extracted with a water- immiscible organic solvent, e.g., butanol and ethyl acetate, thereby passing the active components into the organic layer. SF2582 A, B, and C can further be purified by column chromatography on an adsorbent, e.g., silica gel ("Wako Gel C-200" made by Wako Pure Chemical Inds., Ltd.) and alumina, Sephadex LH-20 (made by Pharmacia Co.), etc.

The present invention is now illustrated in greater detail by way of the following Examples and Test Examples, but it should be understood that the present invention is not deemed to be limited thereto.

## EXAMPLE 1

A seed culture medium comprised 1.0% of starch, 1.0% of glucose, 1.0% of wheat germ, 1.0% of Pharmamedia, 0.2% of Staminol (produced by Sapporo Breweries Ltd.), 1.0% of soybean flour, 0.2% of sodium chloride, 0.2% of dipotassium hydrogenphosphate, and 0.05% of magnesium sulfate.

A production culture medium comprised 5.0% of starch maltose syrup, 0.5% of gluten meal, 1.0% of soybean flour, 0.5% of meat extract, 0.2% of Staminol, 0.1% of magnesium sulfate heptahydrate, 0.2% of sodium chloride, and 0.001% of cobalt chloride hexahydrate.

Before sterilization, the seed culture medium and the production culture medium were adjusted to a pH of 7.0 and 6.5, respectively.

A 100 ml-volume Erlenmeyer flask containing 20 ml of the seed medium was sterilized at 120 °C for 30 minutes, and the medium was inoculated with 2 to 3 (platinum) loopfuls of a slant agar culture of Streptomyces sp. SF2582 (FERM BP-2087) and shake-cultured at 28 °C for 3 days to prepare a first seed culture.

A 500 ml-volume Erlenmeyer flask containing 80 ml of the seed medium was sterilized at 120 °C for 30 minutes, and 2.4 mℓ of the above prepared first seed culture was inoculated thereon, followed by shake-culturing at 28 °C for 1 day to obtain a second seed culture.

A 5 ℓ-volume Erlenmeyer flask containing 1 liter of the seed culture medium was sterilized at 120 °C for 30 minutes and inoculated with 30 ml of the second seed culture, followed by shake-culturing at 28 °C for 1 day to prepare a third seed culture.

Each of five tanks (300-ℓ volume) each containing 200 ℓ of the production medium having been sterilized at 120 °C for 30 minutes was inoculated with 2 ℓ of the third seed culture and cultivated at 28 °C for 5 days under aeration (100 ℓ/min) and shaking (100 rpm in the initial stage of cultivation; 130 rpm after 24 hours from the start of cultivation).

After completion of the cultivation, diatomaceous earth was added to the culture as a filtration aid, and the culture was filtered to recover a cake containing the cells.

## EXAMPLE 2

The cake obtained in Example 1 was extracted with 350 ℓ of a 60% acetone aqueous solution. The extract containing active components was concentrated under reduced pressure to a volume of 100 ℓ, and the concentrate was extracted with 100 ℓ of ethyl acetate. The active component-containing extract was concentrated under reduced pressure to obtain 240 g of an oily substance. One liter of n-hexane was added thereto, and the hexane-soluble matter was removed. The hexane-insoluble matter was dried to obtain 102 g of an oily substance, which was then passed through a column packed with 3 ℓ of silica gel (Wako Gel C-200), washed with 4 ℓ of chloroform, and developed with 7 ℓ of a mixed solvent of chloroform and methanol (50:1) to collect 500 mℓ fractions. Fraction Nos. 3 to 11 containing SF2582 A and B were combined and concentrated to obtain 16 g of an oily substance. The oily substance (16 g) was passed through a column of 800 mℓ of silica gel (Wako Gel C-200), washed successively with 1 ℓ of benzene-acetone (20:1) and 2.5 ℓ of benzene-acetone (10:1), and then developed with 2.5 ℓ of a mixed developing solvent of benzene-acetone (5:1) to collected 30 mℓ fractions. Fraction Nos. 12 to 52 containing SF2582 A and B were combined and concentrated to dryness under reduced pressure to obtain 2.63 g of a crude yellowish brown powder.

The powder (2.63 g) was dissolved in a small amount of methanol, and the solution was passed through a column packed with 2.0 ℓ of Sephadex LH-20 using methanol and developed with methanol to collect 30 ml fractions. Fraction Nos. 78 to 92 containing SF2582 B were combined and concentrated to dryness under reduced pressure to recover 208 mg of SF2582 B (purity: about 50%). Fraction Nos. 95 to 124 containing SF2582 A were combined and concentrated to dryness under reduced pressure to recover 422 mg of SF2582 A (purity: about 45%).

The crude powder of SF2582 A (422 mg) was subjected to preparative layer chromatography ("Art 5717" produced by E. Merck, Darmstadt) and developed with chloroform-methanol (20:1). A yellow band in the vicinity of Rf = 0.6 was scraped off, extracted with ethyl acetate, followed by filtration. The filtrate was concentrated to dryness to obtain 256 mg of SF2582 A as an orange powder. then, the powder was dissolved in 12 mℓ of warm methanol. On standing at 5° C for 1 day, there was precipitated SF2582 A, which was collected by filtration and dried to finally obtain 182 mg of SF2582 A as a yellowish orange powder.

Similarly, the crude powder of SF2582 B (208 mg) was subjected to preparative layer chromatography ("Art 5744" produced by E. Merck, Darmstadt) and developed with chloroform-methanol (20:1). A yellow band in the vicinity of Rf = 0.6 was scraped off, extracted with ethyl acetate, and filtered. The filtrate was concentrate to dryness under reduced pressure to obtain 124 mg of SF2582 B. The product was again subjected to preparative layer chromatography using benzene-acetone (5:2) as a developing solvent to obtain 92 mg of SF2582 B. The resulting product (92 mg) was dissolved in a small amount of methanol. and the solution was passed through a column packed with 350 mℓ of "Toyopearl HW-40" (produced by Tosoh Corporation) using methanol and developed with methanol to collect 10 mℓ fractions. Yellowish orange fraction of from Nos. 46 to 54 containing SF2582 B were combined and concentrated to dryness to recover 88 mg of SF2582 B as a yellowish orange powder.

## EXAMPLE 3

The cell cake obtained in Example 1 was extracted with 350 ℓ of a 60% acetone aqueous solution. The extract containing the active components was concentrated under reduced pressure to obtain a 100 ℓ of an aqueous solution, which was then extracted with 100 ℓ of ethyl acetate. The ethyl acetate extract was concentrated under reduced pressure to obtain 222 g of an oily substance. To the oily substance was added 1 ℓ of n-hexane, and the hexane-soluble matter was removed. The hexane-insoluble matter was dried to obtain 96 g of an oily substance. The product was passed through a column containing 3 ℓ of silica gel (Wako Gel C-200), washed with 4 ℓ of chloroform, and developed with a mixed solvent of chloroform-methanol (50:1) (10 ℓ) to collect 500 mℓ fractions. Active fraction Nos. 12 to 16 containing SF2582 C were combined and concentrated to precipitate yellowish orange crystals, which were collected by filtration and dried to obtain 920 mg of crude crystals of SF2582 C. The resulting crude crystals (920 mg) were recrystallized twice from a mixed solvent of chloroform-methanol (5:1) to obtain 450 mg of SF2582 C as orange crystals.

## TEST EXAMPLE 1

### Antimicrobial Activity of SF2582 A and B

The antimicrobial activity of SF2582 A and B on various test microorganisms shown in Table 2 below were determined by the serial dilution method on agar plate, and the minimum inhibitory concentrations (µg/ml) obtained are shown in Table 2 below.

TABLE 2

| Test Microorganism | Minimum Inhibitory Concn. (μg/mℓ) | |
|---|---|---|
| | SF2582 A | SF2582 B |
| *Staphylococcus aureus* 209P JC-1 | <0.025 | 0.05 |
| *Staphylococcus aureus* Smith S-424 | <0.025 | <0.025 |
| *Staphylococcus aureus* No. 26 | <0.025 | <0.025 |
| *Staphylococcus epidermidis* ATCC 14990 | 0.05 | 0.05 |
| *Staphylococcus epidermidis* 109 | <0.025 | 0.05 |
| *Enterococcus faecalis* ATCC 8043 | 0.05 | 0.10 |
| *Bacillus anthracis* No. 119 | 0.05 | 0.05 |
| *Escherichia coli* JC-2 | 12.5 | 25 |
| *Escherichia coli* No. 29 | 6.25 | 12.5 |
| *Escherichia coli* W 3630 RGN 823 | 6.25 | 12.5 |
| *Escherichia coli* JR 661 W/677 | 12.5 | 25 |
| *Citrobacter freundii* GN 346 | 3.13 | 6.25 |
| *Salmonella typhi* O-901-W | 3.13 | 3.13 |
| *Salmonella enteritidis* No. 11 | 0.78 | 1.56 |
| *Salmonella typhimurium* LT-2 | 12.5 | 25 |
| *Salmonella* sp. D-0001 | 12.5 | 25 |

/To be cont'd.

TABLE 2 (cont'd.)

| Test Microorganism | Minimum Inhibitory Concn. (μg/mℓ) SF2582 A | SF2582 B |
|---|---|---|
| *Shigella sonnei* EW 33 Type 1 | 3.13 | 6.25 |
| *Klebsiella pneumoniae* PCI 602 | 25 | 50 |
| *Klebsiella pneumoniae* 22 #3038 | 25 | >100 |
| *Proteus vulgaris* OX 19 | >100 | 50 |
| *Proteus mirabilis* GN 310 | 25 | 25 |
| *Providencia rettgeri* J-0026 | 25 | 25 |
| *Morganella morganii* Kono | 50 | 50 |
| *Serratia marcescens* MB-3848 | 25 | 25 |
| *Pseudomonas aeruginosa* MB-3829 | 25 | 50 |
| *Manthomonas maltophilia* M-0627 | 100 | 100 |

As can be seen from Table 2, the novel antibiotics of the present invention, SF2582 A and B, exhibit strong antimicrobial activities on various bacteria, particularly Gram positive bacteria.

TEST EXAMPLE 2

Antitumor Activity of SF2582 A and B

SF2582 A or B was intraperitoneally administered to mice in which P-388 tumor cells had been intraperitoneally implanted at a dose shown in Table 3, once a day for consecutive 2 days or only once, respectively. The effect of improving incidence of survival of animals (T/C %) is shown in Table 3 blow.

TABLE 3

| SF2582 A | | SF2582 B | |
|---|---|---|---|
| Dose (mg/kg) | T/C (%) | Dose (mg/kg) | T/C (%) |
| 2.14 | 80 | 10.0 | 80 |
| 0.54 | 128 | 2.5 | 159 |
| 0.13 | 148 | 0.63 | 159 |
| 0.033 | 114 | 0.16 | 117 |

It is apparent from Table 3 that SF2582 A and B exhibit strong antitumor activity on leukosarcoma P-388 in mice.

TEST EXAMPLE 3

Antitumor Activity of SF2582 C

Cytotoxicity of SF2582 C on various test tumor cells shown in Table 4 below was determined as follows. After SF2582 C was added to a medium, the test tumor cells were cultured at 37° C for 72 hours in 5% $CO_2$. After the culturing, a 50% inhibitory concentration ($IC_{50}$) of SF2582 C on the cells was calculated by the probit method. The results obtained are shown in Table 4.

TABLE 4

| Tumor Cells | Origin | $IC_{50}$(μg/mℓ) |
|---|---|---|
| P-388 | mouse leukemia | 9.4 |
| P-388/ADR | mouse leukemia resistant to multiple drugs | 14.5 |
| L-1210 | mouse leukemia | 1.3 |
| L-1210/CPR | mouse leukemia resistant to Cisplatın | 7.9 |
| Meth-A | mouse fibrosarcoma | 42.0 |
| B16 | mouse melanoma | 5.1 |
| K562 | human leukaemia | 35.6 |
| PC10 | human lung cancer | 14.1 |
| PC13 | " | 39.5 |
| PC14 | " | >50.0 |
| HMV-1 | human melanoma | 12.1 |
| KB | human nasopharynx | 18.0 |
| HL-60 | human leukemia | 31.5 |

It can be seen from Table 4 that SF2582 C exhibits cytoxicity on various tumor cells.

TEXT EXAMPLE 4

Acute Toxicity of SF 2582 A, B, and C

SF2582 A or B was intraperitoneally administered to mice, and the animals were observed for 2 weeks. As a result, $LD_{50}$ of SF2582 A was 0.25 to 1.0 mg/kg and that of SF2582 B was 1.0 to 2.0 mg/kg. In the acute toxicity test of SF2582 C in mice, there was no case of death at a dose of 200 mg/kg (i.p.).

While the invention has been described in detail and with reference to specific embodiments thereof, it will be apparent to one skilled in the art that various changes and modifications can be made therein without departing from the spirit and scope thereof.

## Claims

1. An antibiotic substance produced by a microorganism belonging to the genus Streptomyces (FERM BP-2087) by culturing said microorganism in a medium suitable for growth thereof at 26 to 30 °C for 2 to 7 days under aeration.

2. The antibiotic substance of Claim 1 which is selected from the group consisting of SF2582 A represented by the formula (A)

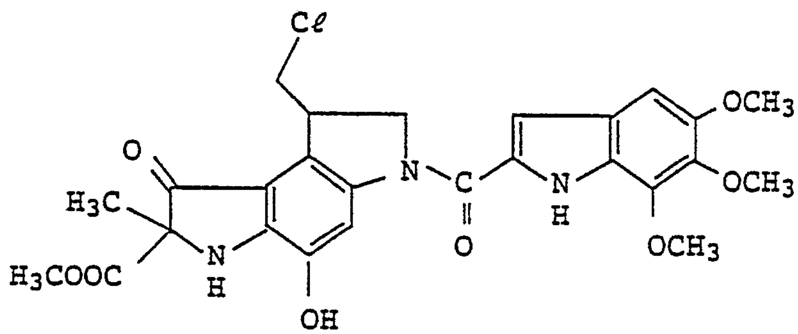

(A)

SF2582 B represented by the formula (B)

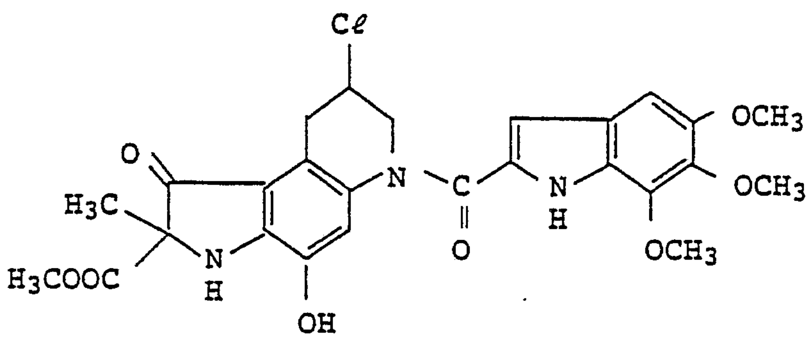

(B)

and SF2582 C represented by the formula (C)

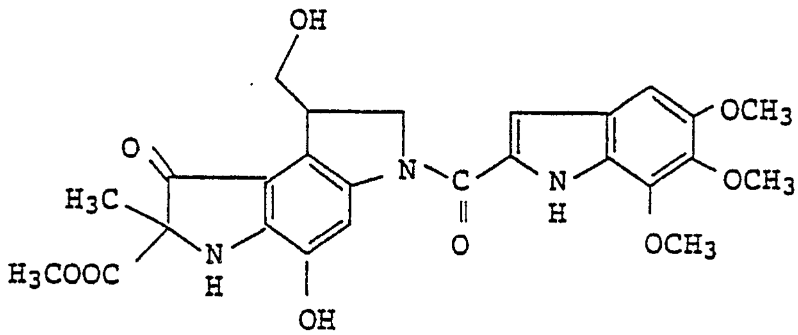

(C)

3. The antibiotic substance of Claim 2, wherein SF2582 A represented by the formula (A)

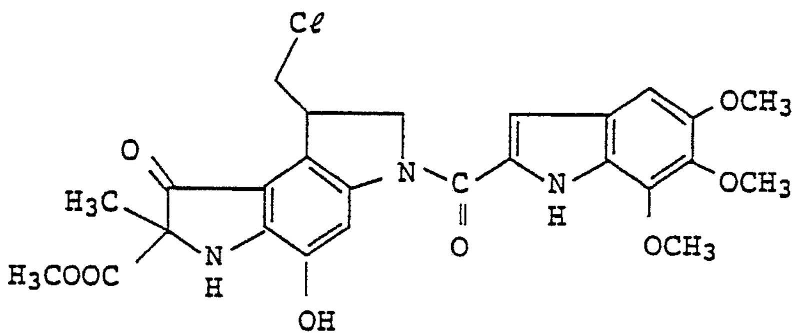

(A)

has the following physiochemical properties:

1) Appearance: yellowish orange amorphous powder

2) Melting Point: 235 - 237 °C (decomp.)

3) Molecular Formula: $C_{26}H_{26}N_3O_8C\ell$ [determined by high resolution mass spectrometry of a monoacetyl derivative; found: 585.1492; calcd. for $C_{28}H_{28}N_3O_9C\ell$: 585.1512]

4) Mass Spectrum: SI-MS m/z 544 $(M+1)^+$
FD-MS m/s 543 $(M)^+$

5) Specific Rotation: $[\alpha]_D^{22}$ -51° (c = 0.2, methanol)

6) UV Spectrum; as shown in Fig. 1
$\lambda_{max}$ (nm) ($E^{1\%}_{1cm}$) (in methanol solution): 208 (729), 245 shoulder (360), 298 (391), 337 (580), 432 (77)

7) IR Spectrum: as shown in Fig. 2

8) $^1$H NMR Spectrum (400 MHz, $CDC\ell_3$): as shown in Fig. 3

9) $^{13}$C NMR Spectrum (100 MHz, $CDC\ell_3$): as shown in Fig. 4 $\delta$ (ppm): 196.6 (s), 169.5 (s), 160.5 (s), 150.2 (s), 150.1 (s), 144.1 (s), 140.7 (s), 138.6 (s), 137.6 (s), 129.0 (s), 125.9 (s), 123.4 (s), 119.5 (s), 115.5 (d), 112.4 (s), 107.8 (d), 97.7 (d), 71.1 (s), 61.5 (q), 61.2 (q), 56.3 (q), 54.9 (t), 53.3 (q), 46.3 (t), 42.2 (d), 21.9 (q)

10) Solubility: soluble in methanol, ethyl acetate, acetone, chloroform, or dimethyl sufoxide; insoluble in water or haxane

11) Thin Layer Chromatography (on silica gel plates produced by E. Merck, Darmstadt):

| Developing Solvent | Rf |
|---|---|
| chloroform-methanol (20:1) | 0.52 |
| benzene-acetone (2:1) | 0.48 |
| toluene-acetone (2:1) | 0.45 |

12) Color Reaction: positive with 10% solfuric acid reagent, Lemieux reagent, Greig-Leaback reagent, and molybdate-sulfuric acid reagent; negative with ninhydrin reagent

4. The antibiotic substance of Claim 2, wherein SF2582 B represented by the formula (B)

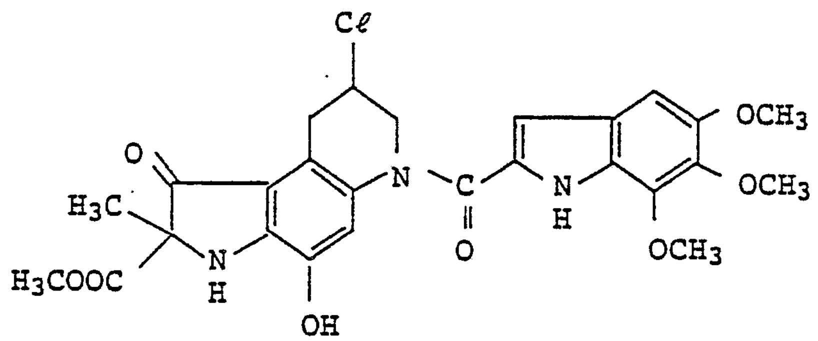

(B)

has the following physiochemical properties:

1) Appearance: yellowish orange amorphous powder

2) Melting Point: 173 - 175 °C (decomp.)

3) Molecular Formula: $C_{26}H_{26}N_3O_8Cl$ [determined by high resolution mass spectrometry of a monoacetyl derivative; found: 585.1519; calcd. for $C_{28}H_{28}N_3O_9Cl$: 585.1512]

4) Mass Spectrum: SI-MS 544 $(M+1)^+$
EI-MS 543 $(M)^+$

5) Specific Rotation: $[\alpha]_D^{22}$ -128° (c=0.2, methanol)

6) UV Spectrum: as shown in Fig. 5
$\lambda_{max}$ (nm) ($E_{1\,cm}^{1\,\%}$) (in methanol solution): 210 (991), 320 (563), 400-410 (99)

7) IR Spectrum: as shown in Fig. 6

8) $^1$H NMR Spectrum (400 MHz, $CDCl_3$): as shown in Fig. 7

9) $^{13}$C NMR Spectrum (100 MHz, $CDCl_3$): as shown in Fig. 8 δ (ppm): 196.8 (s), 169.6 (s), 164.7 (s), 151.6 (s), 150.0 (s), 141.6 (s), 140.2 (s), 138.8 (s), 129.1 (s), 128.8 (s), 125.9 (s), 123.0 (s), 118.0 (d), 116.7 (s), 116.6 (s), 108.1 (d), 97.6 (d), 71.0 (s), 61.4 (q), 61.1 (q), 56.2 (q), 53.6 (d), 53.4 (q), 52.5 (t), 33.1 (t), 21.8 (q)

10) Solubility: soluble in methanol, ethyl acetate, acetone, chloroform, or dimethyl sufoxide; insoluble in water or haxane

11) Thin Layer Chromatography (on silica gel plates produced by E. Merck. Darmstadt):

| Developing Solvent | Rf |
|---|---|
| chloroform-methanol (20:1) | 0.50 |
| benzene-acetone (2:1) | 0.45 |
| toluene-acetone (2:1) | 0.44 |

12) Color Reaction: positive with 10% sulfuric acid reagent, Lemieux reagent, Greig-Leaback reagent. and molybdate-sulfuric acid reagent; negative with ninhydrin reagent

5. The antibiotic substance of Claim 2, wherein the SF2582 C represented by the formula (C)

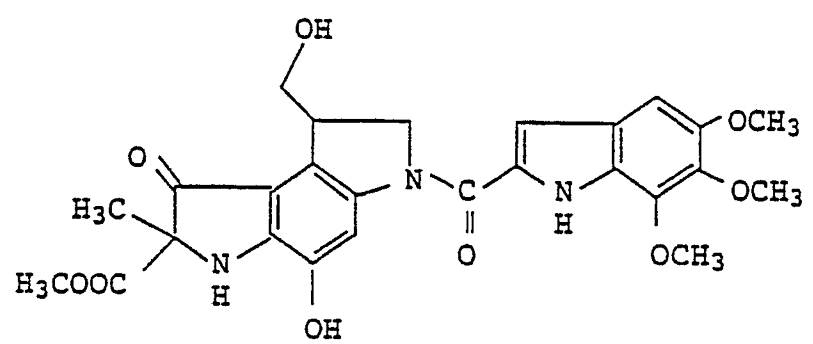

(C)

has the following physiochemical properties:

1) Appearance: orange crystalline powder

2) Melting Point: > 240° C

3) Elementary Analysis: C 59.24%, H 5.12%, N 7.98%

4) Molecular Formula: $C_{26}H_{27}N_3O_9$

5) Mass Spectrum: EI-MS m/z 525 ($M^+$)

6) Specific Rotation: $[\alpha]_D^{22}$ -54° (c = 0.1, methanol)

7) UV Spectrum: as shown in Fig. 9

$\lambda_{max}$ (nm) ($E^{1\%}_{1cm}$) (in methanol solution): 208 (845), 245 shoulder (450), 297 (473), 336 (690), 432 (93)

8) IR Spectrum: as shown in Fig. 10

9) $^1$H NMR Spectrum (400 MHz, DMSO-$d_6$): as shown in Fig. 11

10) $^{13}$C NMR Spectrum (100 MHz, DMSO-$d_6$): as shown in Fig. 12

δ (ppm): 198.1 (s), 169.5 (s), 159.6 (s), 150.5 (s), 149.3 (s), 143.1 (s), 139.9 (s), 139.2 (s), 136.5 (s), 131.4 (s), 125.3 (s), 123.4 (s), 120.0 (s), 114.5 (s), 111.4 (d), 105.9 (d), 98.2 (d), 70.1 (s), 62.6 (t), 61.3 (q), 61.1 (q), 56.1 (q), 54.1 (t), 52.8 (q), 42.5 (d), 20.3 (q)

11) Solubility: easily soluble in pyridine or dimethyl sulfoxide; slightly soluble in methanol; sparingly soluble in chloroform, hexane, ethyl acetate, or water

12) Thin Layer Chromatography (on silica gel plates produced by E. Merck, Darmstadt):

| Developing Solvent | Rf |
|---|---|
| chloroform-methanol (10:1) | 0.38 |
| toluene-acetone (1:1) | 0.62 |

13) Color Reaction: positive with 10% sulfuric acid reagent, Lemieux reagent, Greig-Leaback reagent, and molybdate-sulfuric acid reagent; negative with ninhydrin reagent

6. A process for producing an antibiotic substance of Claim 1 comprising
culturing a microorganism belonging to the genus Streptomyces (FERM BP-2087) in a medium suitable for growth thereof at 26 to 30° C for 2 to 7 days under aeration and
recovering said antibiotic substance obtained.

7. The process of Claim 6, wherein said antibiotic substance is recovered by the steps comprising
filtering the culture medium to obtain the microbial cells,
extracting said antibiotic substance with an organic solvent from said microbial cells and
subjecting the extract to silica gel chromatography to recover said antibiotic substance.

Neu eingereicht / Newly filed
Nouvellement déposé

FIG. 1

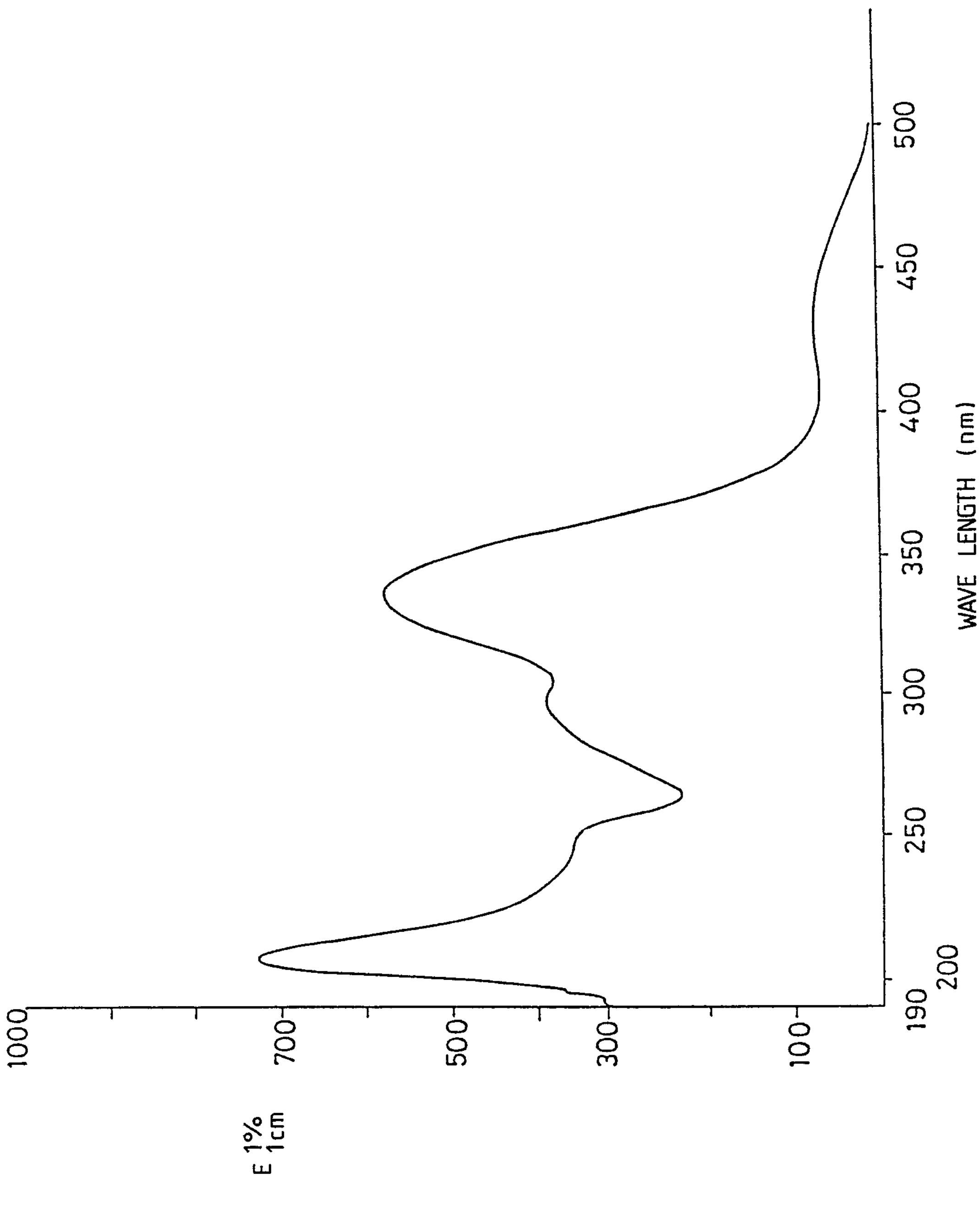

FIG. 2

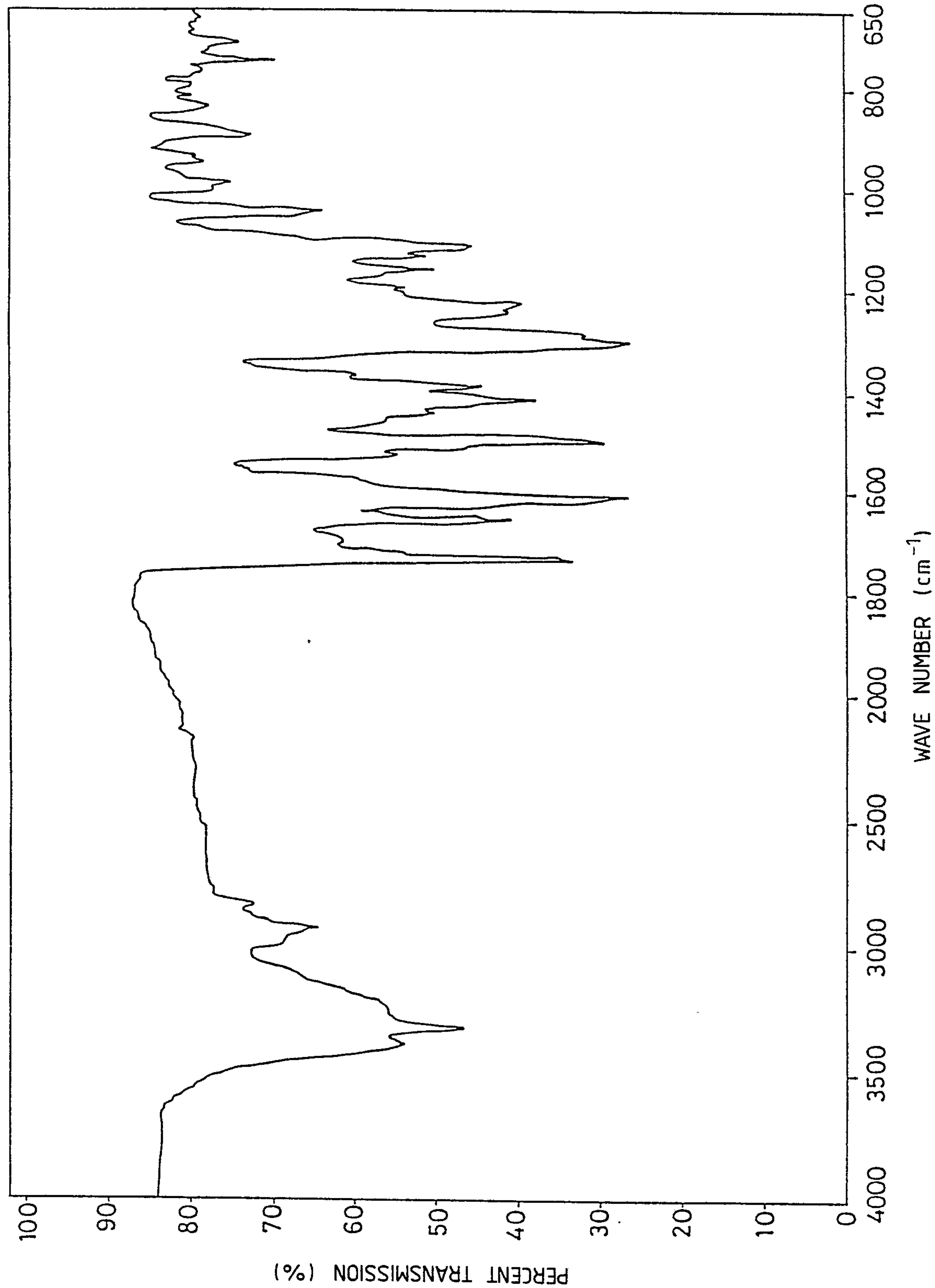
PERCENT TRANSMISSION (%)
100
90
80
70
60
50
40
30
20
10
0
4000
3500
3000
2500
2000
1800
1600
1400
1200
1000
800
650
WAVE NUMBER (cm$^{-1}$)

FIG. 3

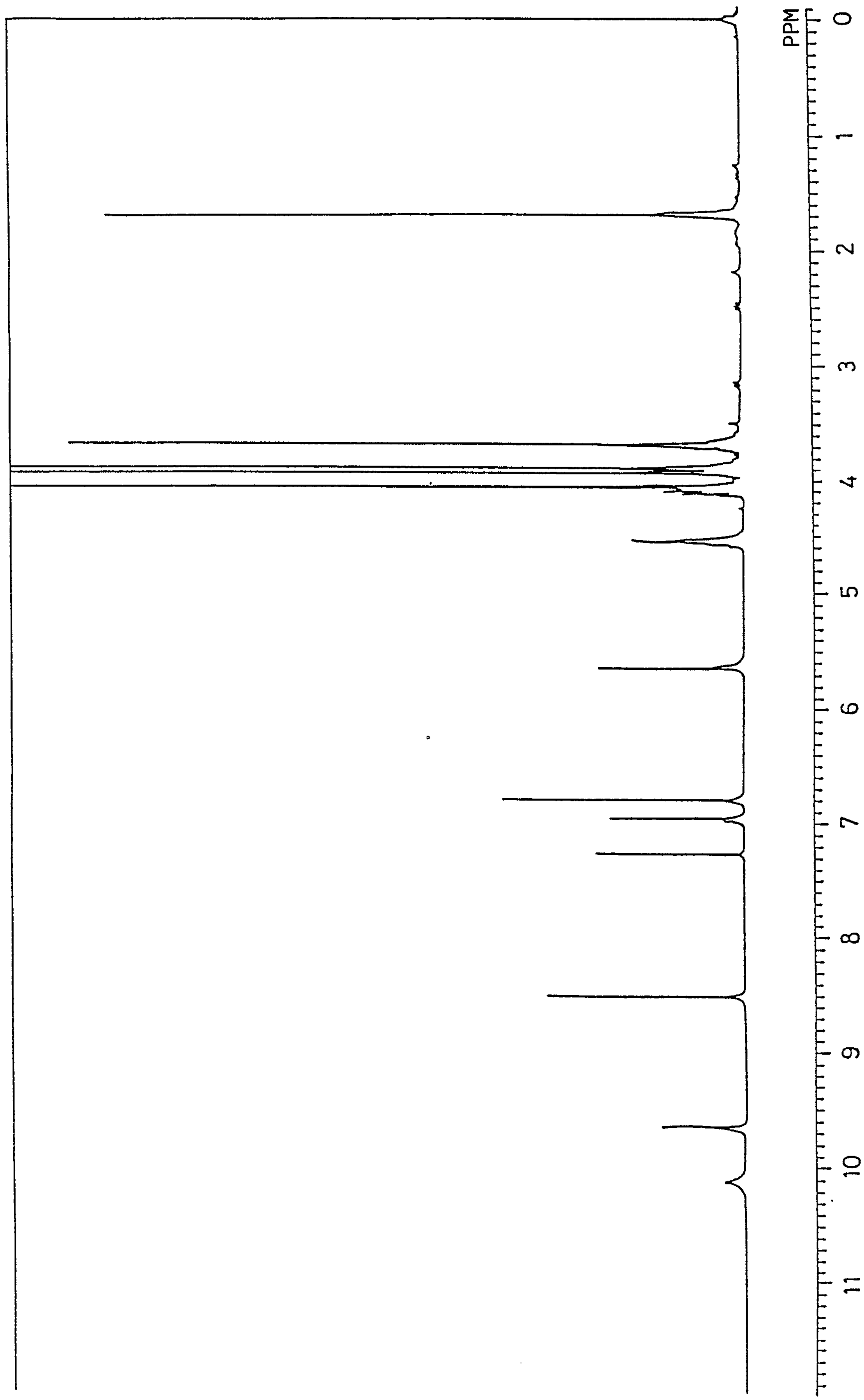

FIG. 4

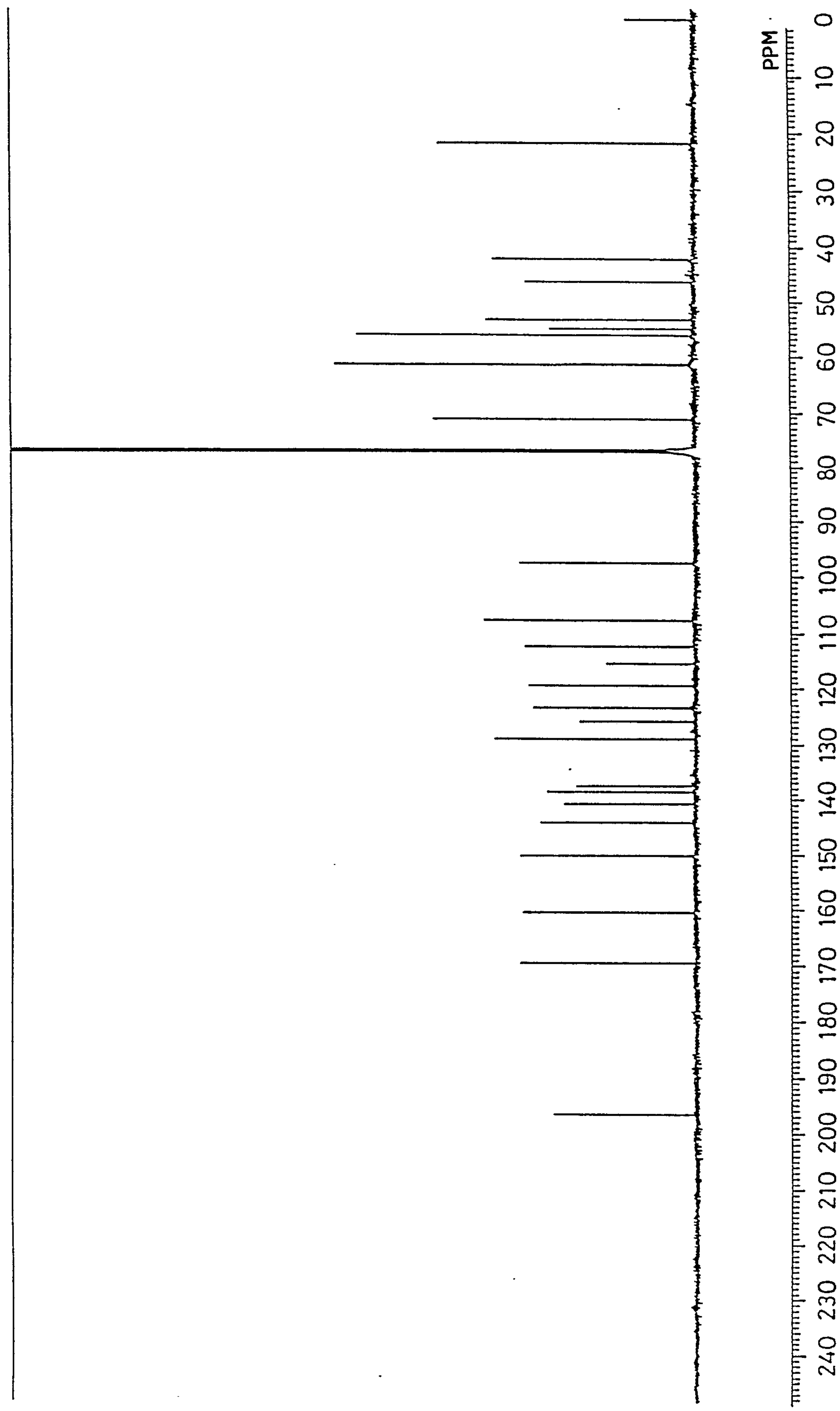
PPM
0
10
20
30
40
50
60
70
80
90
100
110
120
130
140
150
160
170
180
190
200
210
220
230
240

Neu eingereicht / Newly filed
Nouvellement déposé

FIG. 5

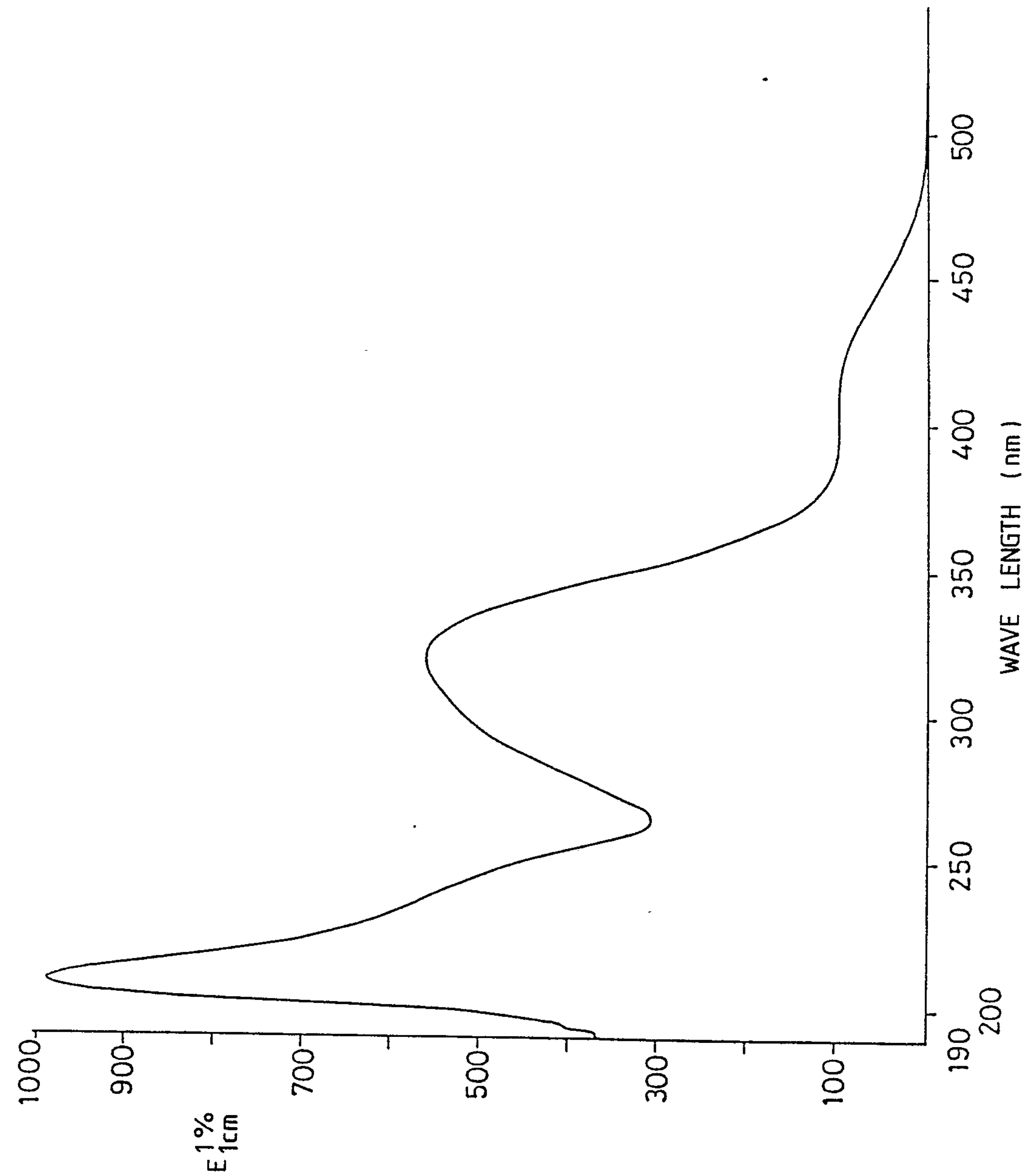

FIG. 6

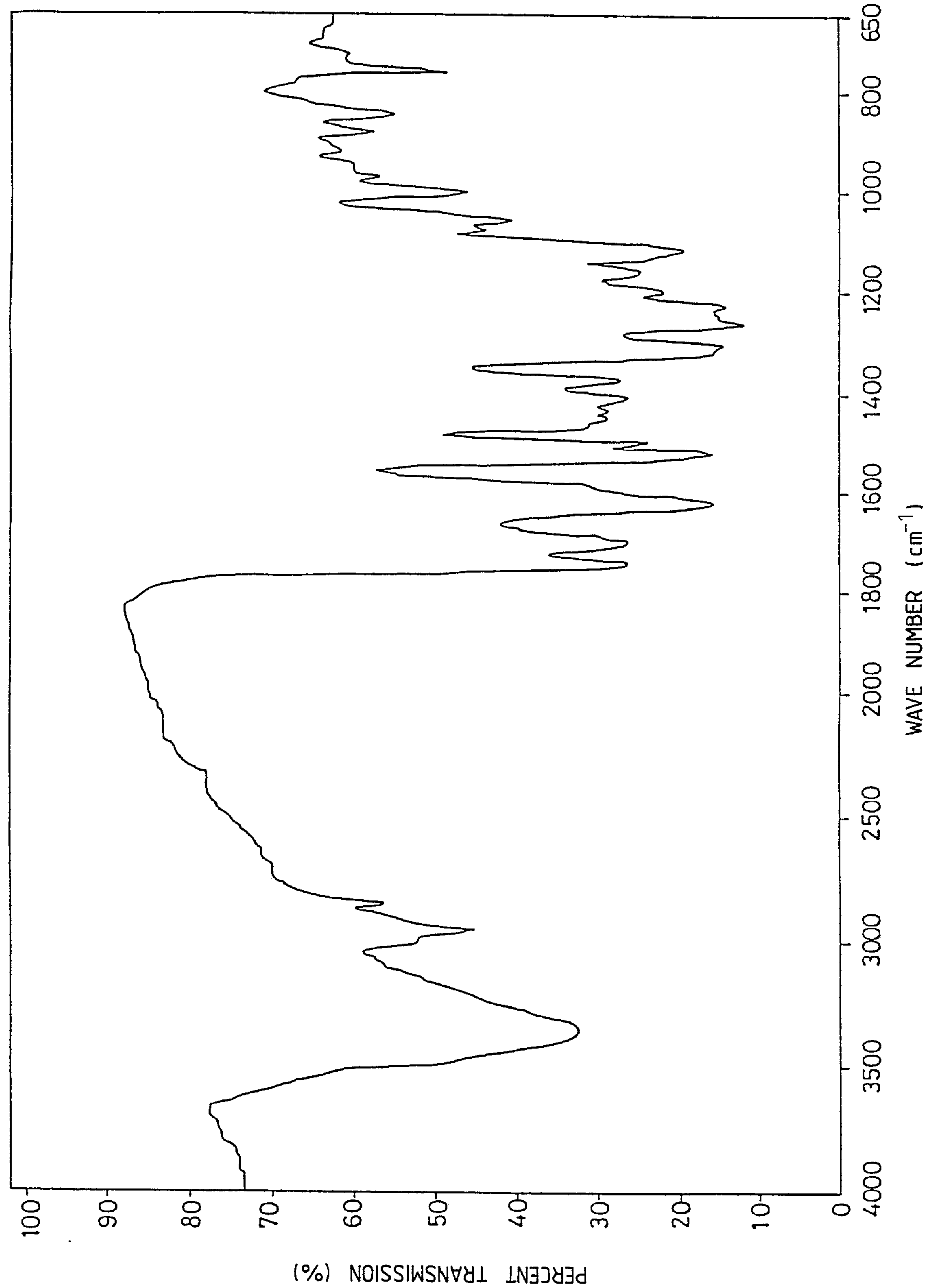
PERCENT TRANSMISSION (%)
0
10
20
30
40
50
60
70
80
90
100
WAVE NUMBER (cm$^{-1}$)
4000
3500
3000
2500
2000
1800
1600
1400
1200
1000
800
650

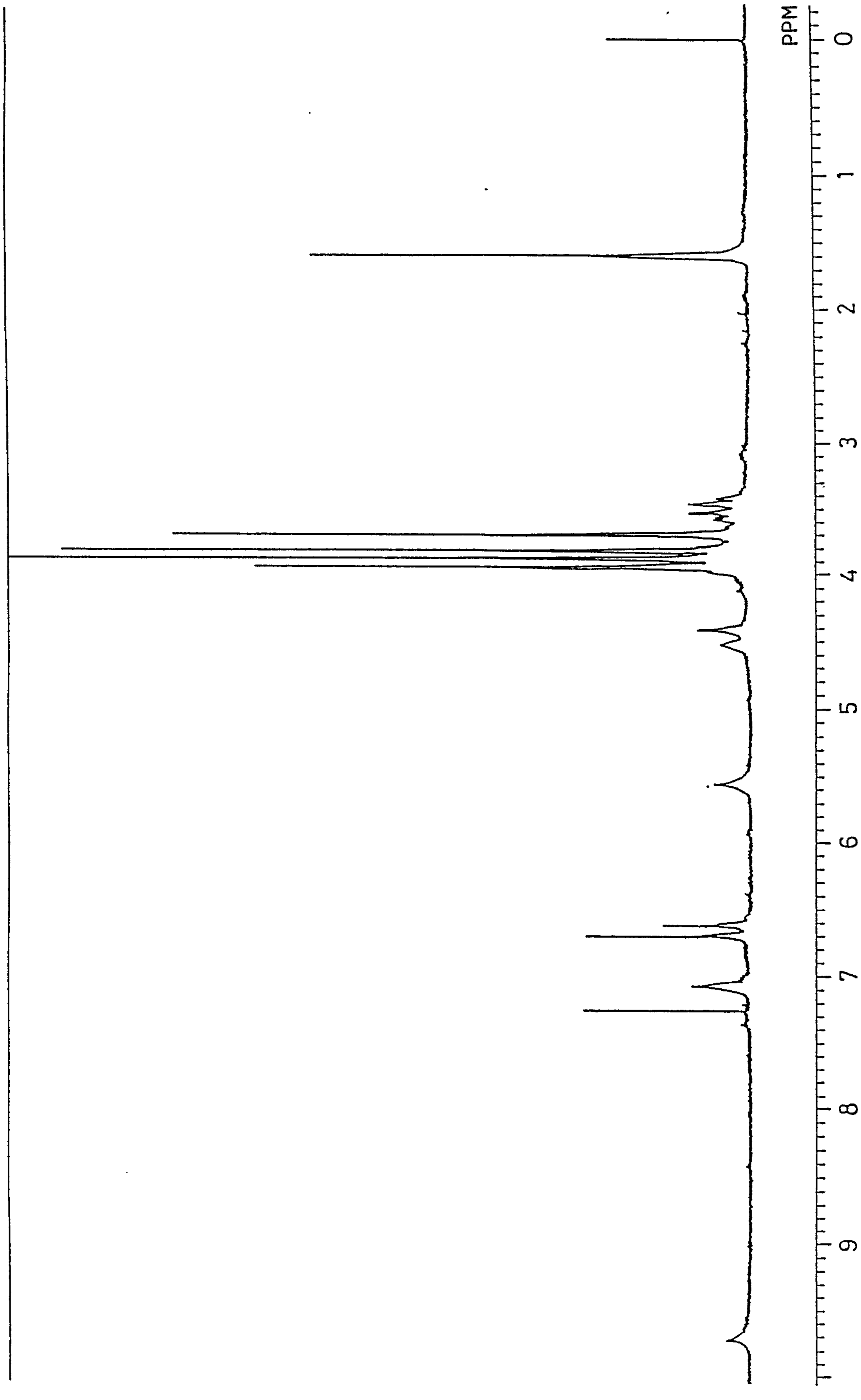


FIG. 7

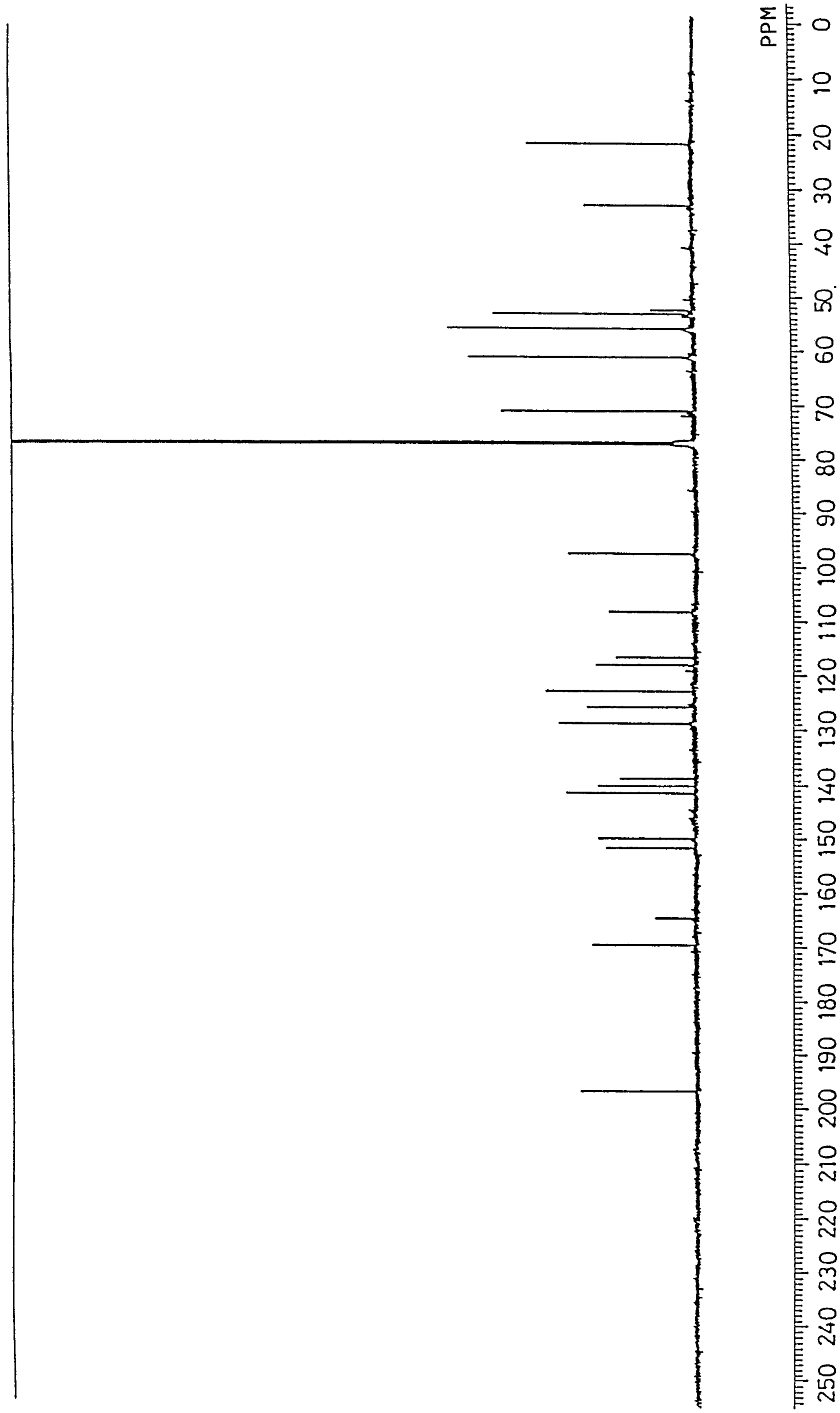
PPM
0
10
20
30
40
50
60
70
80
90
100
110
120
130
140
150
160
170
180
190
200
210
220
230
240
250

FIG. 8

FIG. 9

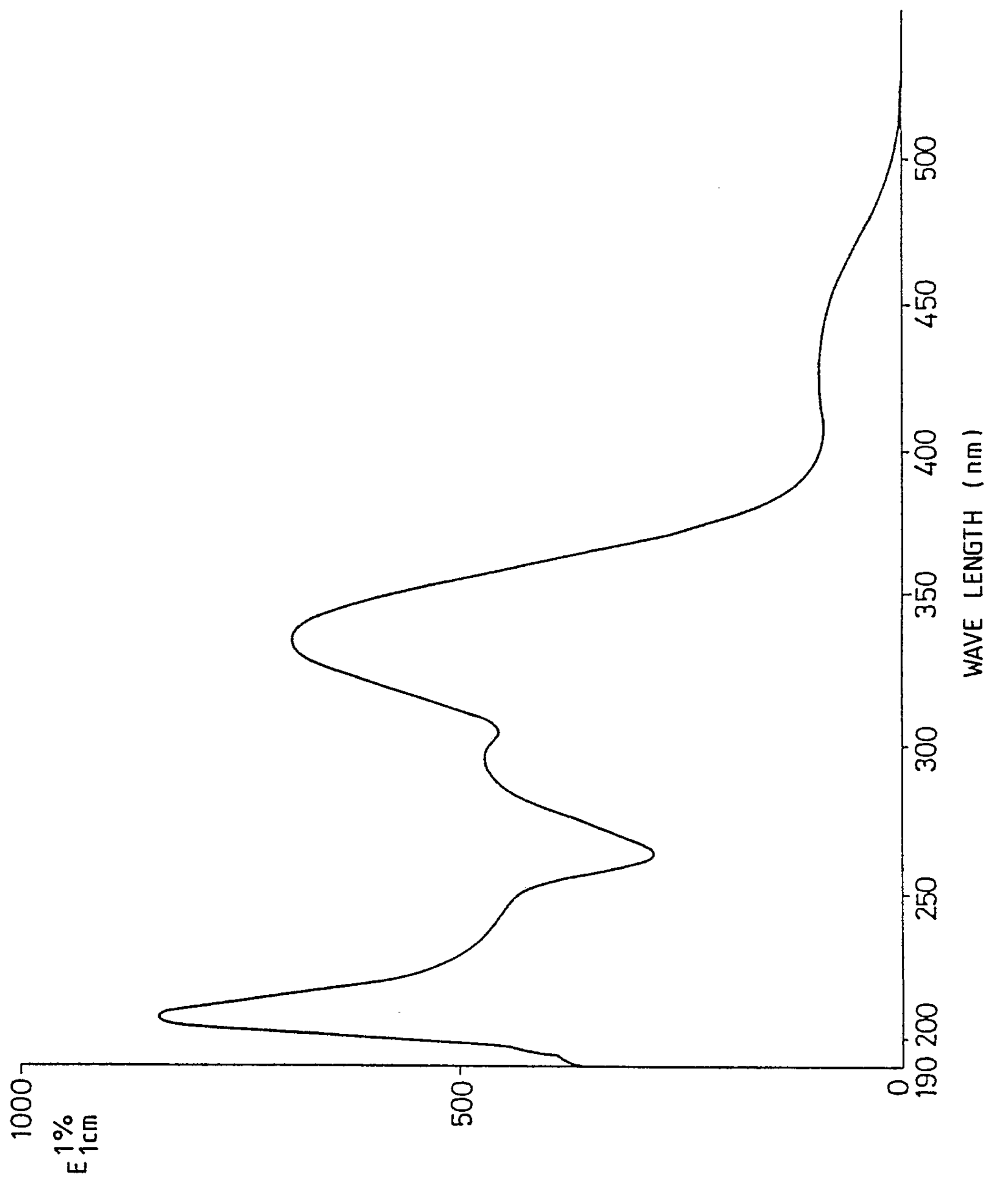

Neu eingereicht / Newly filed
Nouvellement déposé

FIG. 10

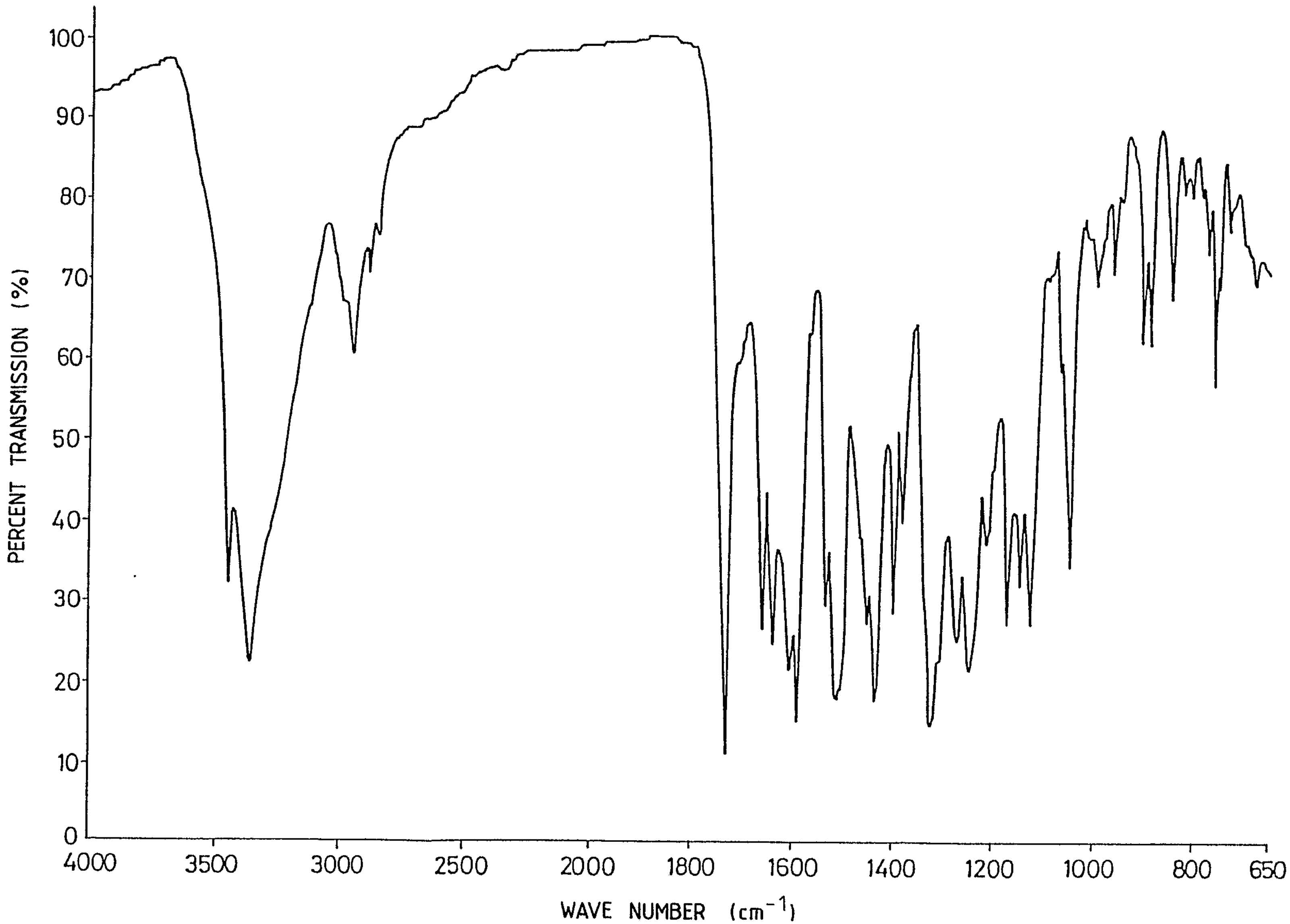

Neu eingereicht / Newly filed
Nouvellement déposé

FIG. 11

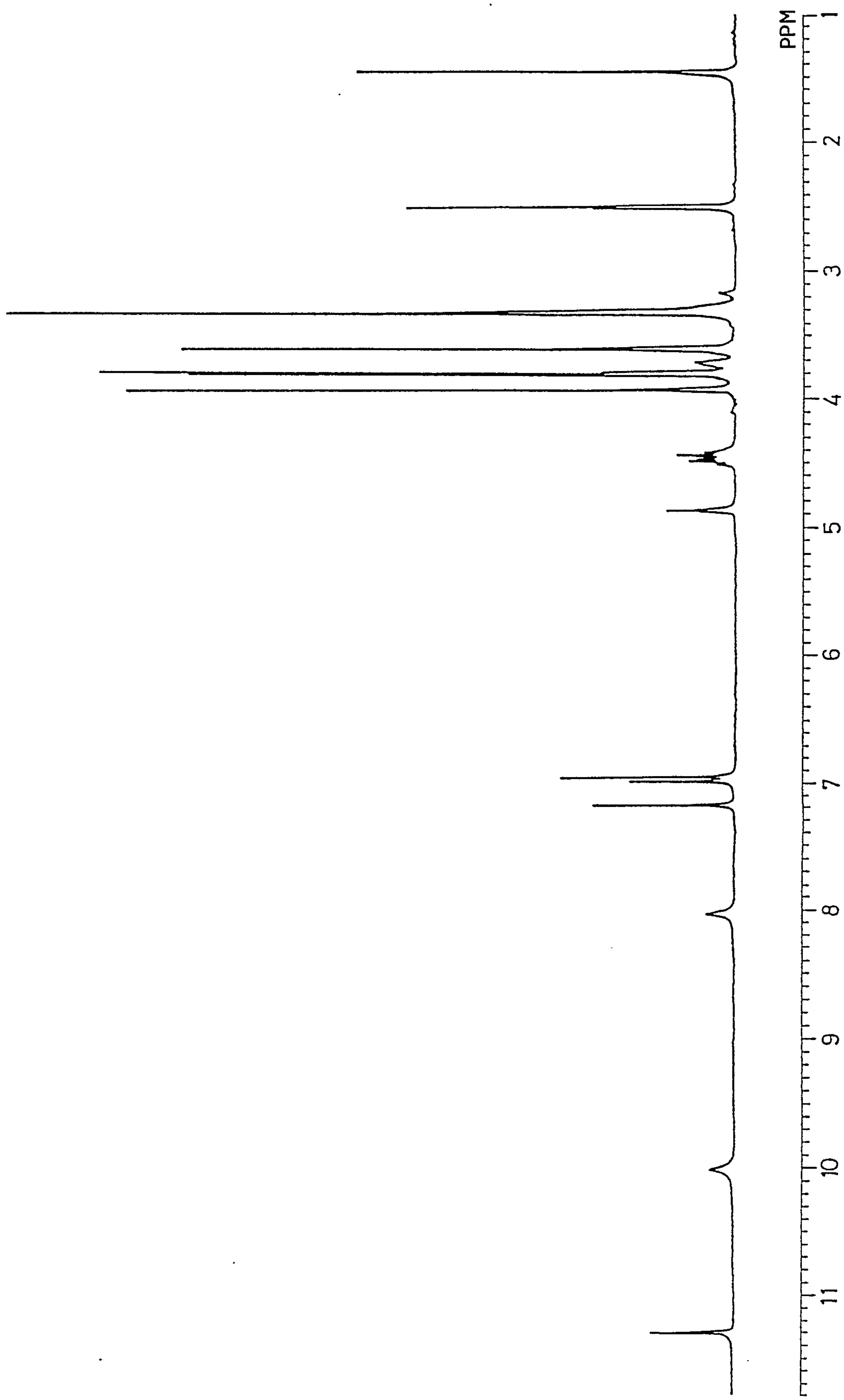

FIG. 12

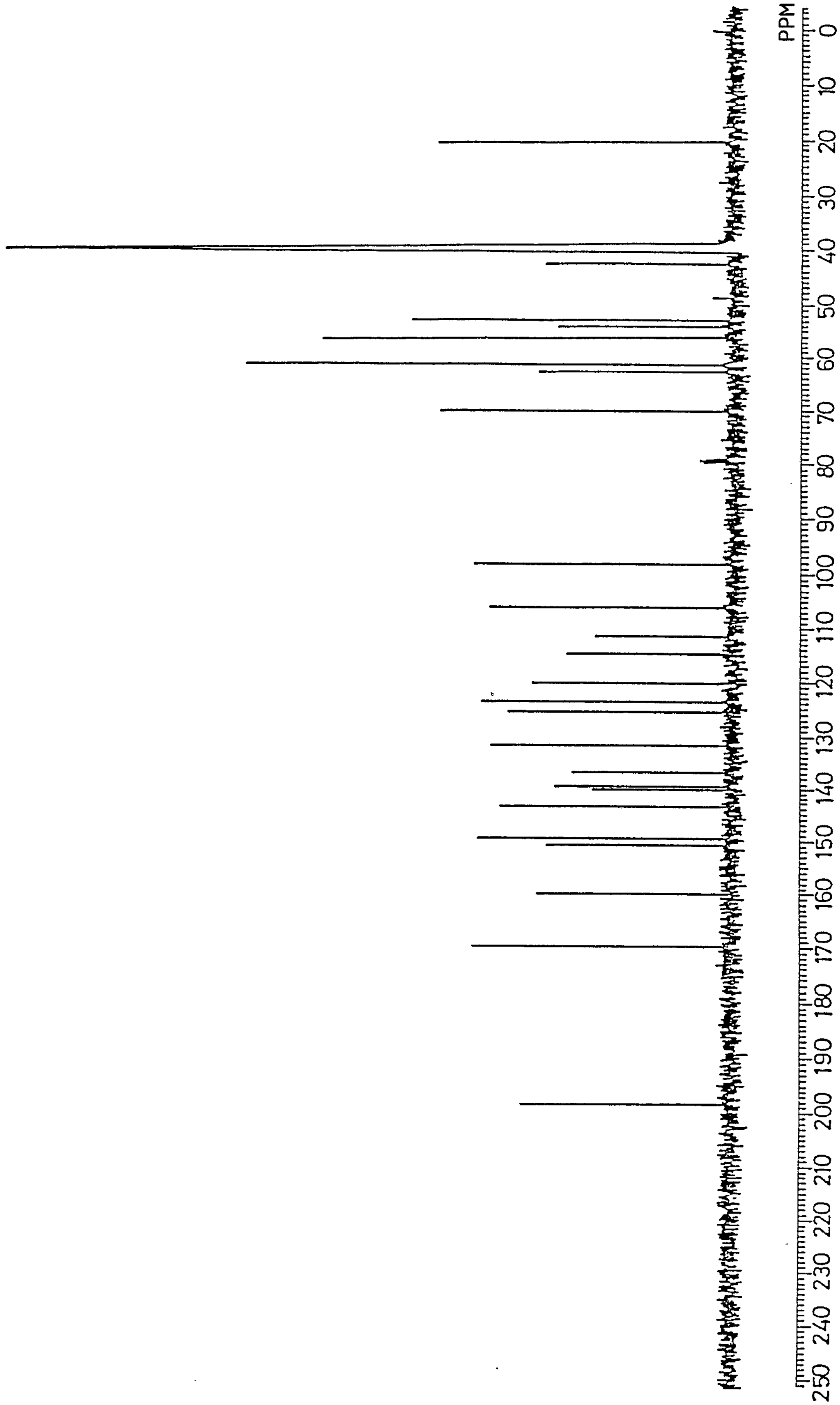
PPM
0
10
20
30
40
50
60
70
80
90
100
110
120
130
140
150
160
170
180
190
200
210
220
230
240
250